# EUROPEAN PATENT SPECIFICATION

(11) **EP 1 429 831 B1**
(45) Date of publication and mention of the grant of the patent: **11.04.2007**
(21) Application number: 02768416.6
(22) Date of filing: 05.08.2002
(51) Int. Cl.: A61M 25/00

(54) **MICROCATHETER WITH IMPROVED DISTAL TIP AND TRANSITIONS**
MIKROKATHETER MIT VERBESSERTER DISTALER SPITZE UND ÜBERGÄNGEN
MICROCATHETER A TRANSITIONS ET A EXTREMITE DISTALE AMELIOREES

(30) Priority: 31.08.2001 US 945225
(43) Date of publication of application: 23.06.2004
(73) Proprietor: Boston Scientific Limited, St. Michael (BB)
(72) Inventor: YE, Ting, Tina, San Jose, CA 95135 (US); MIRIGIAN, Gregory, E., Dublin, CA 94568 (US)
(74) Representative: Vossius & Partner
(86) International application number: PCT/US2002/024692
(87) International publication number: WO 2003/020353

(56) References cited:
- WO-A-99/17826
- US-A- 4 898 591
- US-A- 5 951 539
- US-A- 5 961 511

## Description

The present invention relates generally to intravascular catheters for performing medical procedures. More particularly, the present invention relates to intravascular catheters with improved shaft and distal tip designs. See for example US-A-5 951 539.

### Background of the Invention

Intravascular catheters are used in a wide variety of relatively non-invasive medical procedures. Such intravascular catheters may be used for diagnostic or therapeutic purposes. Generally, an intravascular catheter allows a physician to remotely perform a medical procedure by inserting the catheter into the vascular system of the patient at a location that is easily accessible and thereafter navigating the catheter to the desired target site. By this method, virtually any target site in the patient's vascular system may be remotely accessed, including the coronary, cerebral, and peripheral vasculature.

Typically, the catheter enters the patient's vasculature at a convenient location such as a blood vessel in the neck or near the groin. Once the distal portion of the catheter has entered the patient's vascular system, the physician may urge the distal tip forward by applying longitudinal forces to the proximal portion of the catheter. Frequently the path taken by a catheter through the vascular system is tortuous, requiring the catheter to change direction frequently. In some cases, it may even be necessary for the catheter to bend ninety degrees or more. In order for the catheter to navigate a patient's tortuous vascular system, it is desirable that intravascular catheters be very flexible, particularly near the distal end.

The distance between the access site and the target site is often in excess of 100 cm. The inside diameter of the vasculature at the access site is often less than 2 cm, and the inside diameter of the vasculature at the target site is often less than 0.5 cm. Accordingly, intravascular catheters must be relatively long and thin. Furthermore, in order to navigate through the patient's tortuous vascular system, intravascular catheters must be very flexible. It is also desirable that intravascular catheters be relatively soft in order to minimize the probability of damaging vascular tissue.

Intravascular catheters typically have a radiopaque portion and are guided through the patient's vascular system with the assistance of x-ray fluoroscopy. In this manner, a physician may manipulate the proximal end of the catheter and fluoroscopically monitor the corresponding movement of the distal end of the catheter. As such, it is desirable that intravascular catheters be sufficiently radiopaque along their length and particularly at their distal end such that the physician is able to clearly monitor the progress of the catheter as it is being advanced from the vascular access site to the vascular target site.

After the intravascular catheter has been navigated through the patient's vascular system with the distal end thereof adjacent the target site, the catheter may be used for various diagnostic and/or therapeutic purposes. Frequently, diagnostic and therapeutic techniques require the infusion of fluids through the catheter. For example, it may be desirable to inject radiopaque contrast media through the catheter to provide enhanced fluoroscopic visualization for diagnostic purposes, or to inject pharmaceutical solutions (i.e., drugs) to the target site for therapeutic purposes.

The blood vessels in the brain frequently have an inside diameter of less than 3 mm. Accordingly, it is desirable that intravascular catheters intended for use in these blood vessels have an outside diameter which allows the catheter to be easily accommodated by the blood vessel. The path of the vasculature inside the brain is highly tortuous, and the blood vessels are relatively fragile. Accordingly, it is desirable that the distal portion of a catheter be sized appropriately and be atraumatic for the neurological vasculature.

### Summary of the Invention

The present invention comprises a unique intravascular catheter that incorporates a number of refinements to the shaft and distal tip. According to a preferred embodiment of the invention, a catheter comprises a shaft having a proximal end, a distal end, and a lumen. A hub is typically disposed at the proximal end and a distal tip is disposed at the distal end. The shaft may comprise multiple layers, including an inner liner, a second layer, a third layer, and a fourth layer.

The second layer may be disposed over the inner liner extending from the proximal end of the shaft to a distal terminus. The distal terminus may be about 4 millimeters from the distal end. The absence of the second layer between the distal terminus and the distal end of the shaft improves the physical properties of the catheter. For example, the shaft may be more flexible or generally softer near the distal end, and may be more readily thermoformed.

The third layer may be disposed over the second layer and preferably comprises a coil that is wound over the second layer. The coil may be arranged in a single coil region near the distal end of the shaft. The single coil region is understood to be a single layer of coil wound around the second layer along a longitudinal axis thereof. The coil may further include a multiple coil region near the proximal end of the shaft wherein the coil is wound multiple times around the second layer along the longitudinal axis thereof.

The fourth layer may be disposed over the third layer and may include a taper. Preferably, the taper decreases the diameter of the shaft near the distal end thereof. The decrease in diameter may comprise a suitable reduction in size appropriate for multiple uses of the catheter. For example, a generally small diameter distal tip may be used for procedures involving treatment of relatively small blood vessels.

### Brief Description of the Drawings

Figure 1 is a plan view of an intravascular catheter with an improved shaft, distal tip, and transitions according to a preferred embodiment of the invention;
Figure 2 is an enlarged view of a shaft of the intravascular catheter shown in Figure 1;
Figure 3 is an enlarged view of an alternative shaft of the intravascular catheter shown in Figure 1;
Figure 4 is an enlarged view of another alternative shaft of the intravascular catheter shown in Figure 1; and
Figure 5 is an enlarged view of yet another alternative shaft of the intravascular catheter shown in Figure 1.

### Detailed Description of the Preferred Embodiments

The following description should be read with reference to the drawings wherein like reference numerals indicate like elements throughout the several views. The detailed description and drawings depict select embodiments and are not intended to be limiting.

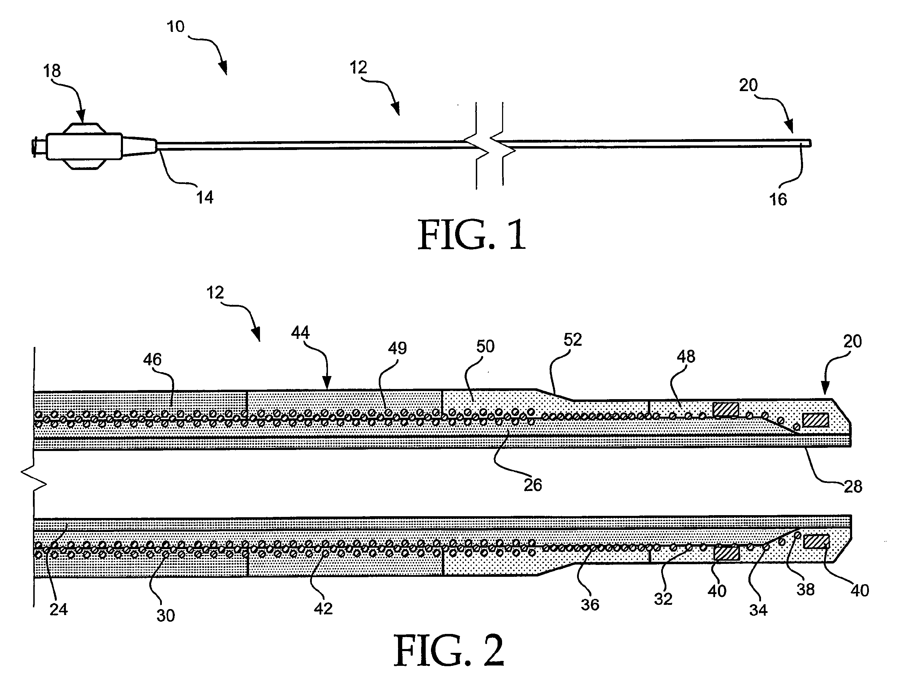

Figure 1 is a plan view of an intravascular catheter 10 with an improved shaft, distal tip, and improved transitions according to a preferred embodiment of the invention. The intravascular catheter 10 comprises a shaft 12 having a proximal end 14 and a distal end 16. A hub 18 is typically disposed at proximal end 14 of shaft 12 and a distal tip 20 having a shapable length is disposed at distal end 16 of shaft 12. Shaft 12 further comprises a lumen 22 as best seen in Figure 2. Lumen 22 may be a guidewire lumen and/or an infusion lumen. Lumen 22 may have a diameter compatible with a guide wire having an outside diameter of about 0,25 mm to 0,35 mm (0.010 to 0.014 inches.)

Shaft 12 comprises multiple layers including an inner liner 24. Preferably, inner liner 24 comprises polytetrafluoroethylene (PTFE). Polytetrafluoroethylene is a preferred material because it creates a smooth, low-friction surface for the passage of other devices or fluids through catheter 10. In an alternate embodiment, inner liner 24 may comprise materials including, but not limited to, thermoplastics, high performance engineering resins, fluorinated ethylene propylene (FEP), polymer, polyethylene (PE), polypropylene (PP), polyvinylchloride (PVC), polyurethane, polyether-ether ketone (PEEK), polyimide, polyamide, polyphenylene sulfide (PPS), polyphenylene oxide (PPO), polysufone, nylon, or perfluoro(propyl vinyl ether) (PFA).

Inner liner 24 may be formed by extrusion over a mandrel. Extrusion may result in inner liner 24 having a thickness of about 0,0125 mm to 0,031 mm (0.0005 inches to .00125 inches) and a diameter of about 0,44 mm to 0,48 mm (0.0175 inches to 0.019 inches) over a length of about 135 cm to 200 cm. In an alternate embodiment, inner liner 24 may be formed by lamination over a mandrel. The mandrel may, for example, comprise nitinol and have a diameter of about 0,41 mm (0.0165 inches). A person of ordinary skill in the art would be familiar with processes and equipment suitable for forming inner liner 24 according to multiple embodiments of the present invention.

A second layer 26 is disposed over inner liner 24. Second layer 26 is comprised of polyether block amide (PEBA). Polyether block amide is commercially available from Atochem Polymers of Birdsboro, Pennsylvania, under the trade name PEBAX. Second layer 26 may comprise PEBAX 55 having a diameter of about 0,46 mm to 0,55 mm (0.0185 inches to 0.022 inches) and a length of about 132 cm to 200 cm.

Second layer 26 extends from proximal end 14 of shaft 12 to a distal terminus 28. Distal terminus 28 is set back from distal end 16 a distance that is equal to or greater than the shapable length of distal tip 20. For example, distal terminus 28 may be 4 millimeters to 3 centimeters from distal end 16 depending on the flexibility and shapable length desired. The absence of second layer 26 between distal terminus 28 and distal end 16 of shaft 12 improves the physical properties of catheter 10. For example, shaft 12 may be more flexible or generally softer near distal end 16, and/or may be more shapable by thermoforming techniques.

Second layer 26 may be formed by securing outer layer 26 near distal end 16 of shaft 12 and laminating to proximal end 14 thereof. Alternatively, second layer 26 may be disposed over inner liner 24 by extrusion.

A third layer 30 is disposed over second layer 26. Third layer 30 comprises a coil manufactured from materials including, but not limited to, stainless steel, metal, nickel alloy, nickel titanium alloy, polymer, round wire, flat wire, magnetic resonance imaging compatible metal, and combinations thereof. A magnetic resonance imaging compatible metal is understood to comprise non-magnetic or non-ferrous metals.

Third layer 30 further comprises a single coil region 32 near distal end 16. The coil may be wound around second layer 26 along a substantial portion of the length thereof. Single coil region 42 is understood to be a single layer of coil wound around second layer 26 along a longitudinal axis thereof, e.g. 0,31 mm (0.0125 inch) outside diameter stainless steel round wire. Third layer 30 further includes a multiple coil region 42 near proximal end 14 of shaft 12 wherein coil is wound multiple times around second layer 26 at a particular point along the longitudinal axis thereof.

Single coil region 32 further comprises a first pitch region 34 and a second pitch region 36. First pitch region 34 comprises a pitch between about 1,25 mm (0.050 inches) per turn and 0,1 mm (0.004 inches) per turn. Second pitch region 36 comprises a pitch between about 0,5 mm (0.020 inches) per turn and 0,05 mm (0.002 inches) per turn. Those skilled in the art will recognize that a number of values may be used to describe the pitch of first pitch region 34 and second pitch region 36 without deviating from the spirit and scope of the invention. For example, first pitch region 34 and second pitch region 36 may be substantially equal.

A distal end 38 of third layer 30 may be secured to a radiopaque marker 40. Preferably, radiopaque markers 40 produce a relatively bright image on a fluoroscopy screen during a medical procedure. This relatively bright image aids the user of catheter 10 in determining the location of distal end 16 of shaft 12. Radiopaque markers 40 may comprise a number of radiopaque materials including, but not limited to, gold, platinum, and plastic material loaded with a radiopaque filler. Catheter 10 may further comprise additional radiopaque markers.

A fourth layer 44 is disposed over third layer 30. Fourth layer 44 comprises polyether block amide (PEBA). Alternately, fourth layer 44 may be comprised of materials similar to those disclosed above, including polymers and metals. Fourth layer 44 may have a length of about 135 cm to 200 cm.

Fourth layer 44 further comprises a proximal end 46, a distal end 48, a first middle section 49, and a second middle section 50. Each individual section of fourth layer 44 may comprise polyether block amide. The durometer of each section may be different. At distal end 48, the preferred material is a low durometer polymer (e.g., PEBAX 2533) to maintain a soft, atraumatic tip. At proximal end 46, the preferred material is a high durometer polymer (e.g., PEBAX 7233) to provide pushability. First middle section 49 and second middle section 50 may provide a smooth transition between proximal end 46 and distal end 48. For example, first middle section 49 may comprise PEBAX 5533 and second middle section 50 may comprise PEBAX 4033. Generally, the durometer decreases from proximal end 46 to distal end 48. Alternatively, fourth layer 44 may be comprised of a single section having a differing durometer on opposite ends.

Fourth layer 44 further comprises a taper 52. Taper 52 decreases the diameter of shaft 12 near distal end 16. Taper 52 may decrease the diameter of shaft 12 to varying degrees. The outside diameter of fourth layer 44 may be about 0,65 mm to 0,88 mm (.026 inches to .035 inches) near proximal end 46 and about 0,53 mm to 0,65 mm (.021 inches to .026 inches) at distal end 48. Preferably, the outside diameter of shaft 12 from taper 52 to distal end 16 is sized appropriately for insertion into generally small blood vessels. For example, distal end 16 may be sized to facilitate entry of shaft 12 into the coronary, peripheral, and neurological vasculature.

Fourth layer 44 may be disposed over third layer 30 by heat fusing separate tube sections 46, 48, 49, and 50 by extrusion. Alternatively, fourth layer 44 is disposed over third layer 30 by lamination.

The combination of layers at distal end 16 of shaft 12 comprises a level of flexibility which makes it unlikely to damage the blood vessels of a patient. According to this embodiment, distal tip 20 is understood to comprise an atraumatic and shapable tip. Moreover, the shapable length of distal tip 20 can be heat set, for example by steam.

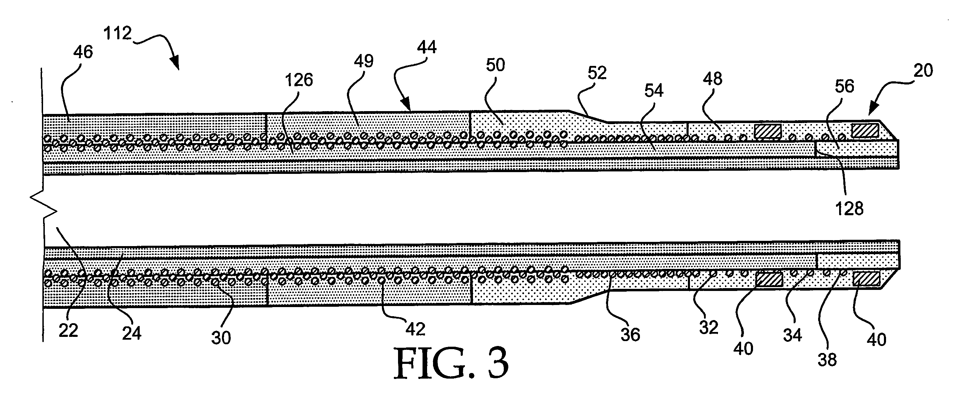

Figure 3 is an enlarged view of an alternate shaft 112 that is essentially similar to shaft 12 with a refinement to second layer 26. Second layer 126 extends from proximal end 14 of shaft 112 to distal terminus 128. Second layer 126 further comprises a second segment 56. Preferably, first segment 54 extends from proximal end 14 of shaft 112 to distal terminus 128 and is substantially similar to second layer 26 as depicted in Figure 2. Second segment 56 preferably extends from distal terminus 128 to distal end 16 of shaft 112. Distal terminus 128 is set back from distal end 16 of shaft 112 a distance equal to or greater than the shapable length of distal tip 20. The durometer of first segment 54 and second segment 56 are different. For example, first segment 54 comprises a generally harder durometer (e.g., PEBAX 5533D) than second segment 56 (e.g. PEBAX 2533D).

Shaft 112 may be manufactured substantially similar to what is disclosed above for shaft 12. A person of ordinary skill in the art would be familiar with alterations in the method of manufacture according to multiple embodiments of the invention.

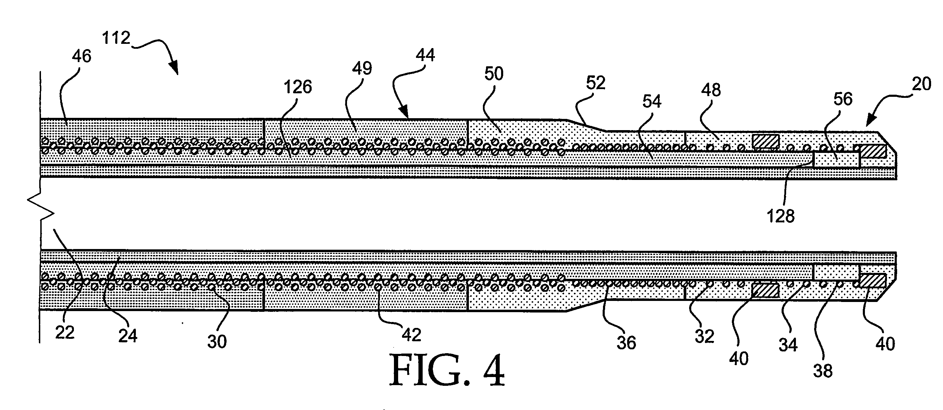

Figure 4 is an enlarged view of an alternate shaft 212 that is essentially similar to shaft 112 with a refinement to second layer 126 and second segment 56. The first segment 54 of the second layer 126 extends from proximal end 14 of the shaft 212 to distal terminus 228. The second segment 56 of the second layer 126 extends from distal terminus 228 to the distal marker band 40 proximal of the distal end 16 of shaft 212. The distal marker band 40 resides within the distal end 48 of the fourth layer 44, which is tapered down to encase the distal marker band 40 and to be connected to the inner layer 24 at the distal end 16 of the shaft 212. Distal terminus 228 is set back from distal end 16 of shaft 212 a distance equal to or greater than the shapable length of distal tip 20. The first segment 54 may have a generally harder durometer (e.g., PEBAX 5533D) than the second segment 56 (e.g. PEBAX 2533D).

Shaft 212 may be manufactured substantially similar to what is disclosed above for shaft 12. A person of ordinary skill in the art would be familiar with alterations in the method of manufacture according to multiple embodiments of the invention.

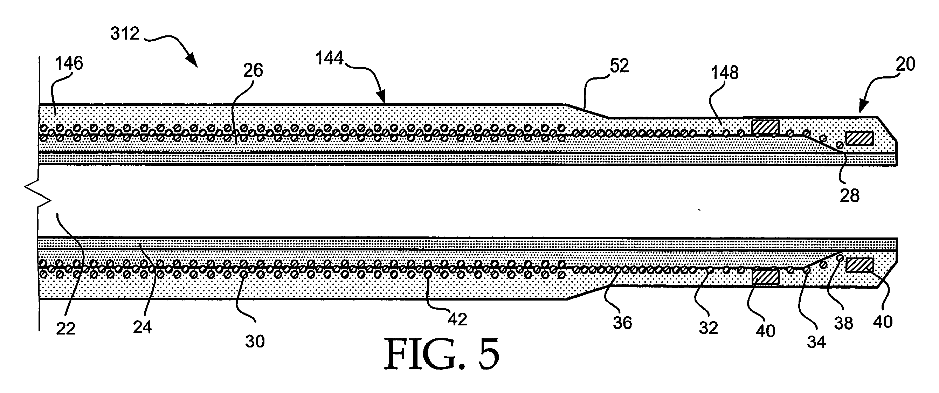

Figure 5 is an enlarged view of an alternative shaft 312 that is essentially similar to shaft 12 with a refinement to fourth layer 44. Fourth layer 144 is disposed over third layer 30. Fourth layer 144 further comprises proximal end 146 and distal end 148. Preferably, fourth layer 144 is comprised of a single layer of PEBA having a differing durometer on opposite ends. For example, the durometer of proximal end 146 may be greater than the durometer of distal end 148. Fourth layer 144 can be disposed over third layer 30 by gradient extrusion. Gradient extrusion is described in U.S. Patent Application Serial No. 09/430,327 to Centell et al., which is hereby incorporated by reference. In summary, gradient extrusion is understood to be an extrusion technique wherein polymers of differing durometer may be disposed onto an object so as to form a smooth transition in a physical property (e.g., durometer). For example, gradient diffusion of fourth layer 144 may result in a generally harder durometer (e.g., PEBAX 7233) near proximal end 146 and a generally softer durometer (e.g., PEBAX 2533) near distal end 148. In addition, gradient diffusion of fourth layer 144 would result in a substantially gradual decrease in durometer from proximal end 146 to distal end 148.

In a preferred embodiment, shaft 312 may be manufactured substantially similar to what is disclosed above for shaft 12. A person of ordinary skill in the art would be familiar with alterations in the method of manufacture according to multiple embodiments of the invention.

Numerous advantages of the invention covered by this document have been set forth in the foregoing description. It will be understood, however, that this disclosure is, in many respects, only illustrative. Changes may be made in details, particularly in matters of shape, size, and arrangement of steps without exceeding the scope of the invention. The invention's scope is, of course, defined in the language in which the appended claims are expressed.

## Claims

1. An intravascular catheter, comprising:
an elongate shaft (12) having a proximal end, a distal end, and a distal tip having a shapable length, the elongate shaft including:
**characterised in that**
an inner liner (24);
a second layer (26) disposed over the inner liner (24), the second layer (26) including a first segment and a second segment, the first segment extending to a distal terminus (28) and the second segment extending from the distal terminus (28) to a radiopaque marker band (40) disposed proximal of the distal end of the shaft, wherein the distal terminus (28) is set back from the distal end of the shaft a distance equal to or greater than the shapable length;
a third layer (30) disposed over the second layer; the third layer (30) including a single coil region near the distal end of the shaft and a multiple coil region near the proximal end of the shaft; and
a fourth layer (44) disposed over the third layer (30), the fourth layer (44) including a proximal end and a distal end, wherein the durometer at the proximal end is greater than the durometer at the distal end.

2. The catheter in accordance with claim 1, wherein the distal terminus (28) is about 4 millimeters from the distal end of the shaft.

3. The catheter in accordance with claim 2, wherein the shape of the distal.tip can be heat set.

4. The catheter in accordance with claim 3, wherein the shape of the distal tip can be heat set by steam.

5. The catheter in accordance with claim 1, wherein the inner liner (24) comprises polytetrafluoroethylene.

6. The catheter in accordance with claim 5, wherein the second layer (26) comprises polyether block amide.

7. The catheter in accordance with claim 6, wherein the third layer (30) comprises a coil.

8. The catheter in accordance with claim 7, wherein the coil comprises stainless steel.

9. The catheter in accordance with claim 7, wherein the coil comprises nickel alloy.

10. The catheter in accordance with claim 7, wherein the coil comprises a non-ferrous metal.

11. The catheter in accordance with claim 7, wherein the fourth layer (44) comprises polyether block amide.

12. The catheter in accordance with claim 11, wherein the distal end of the shaft (12) has an outside diameter that is less than the outside diameter of the proximal end of the shaft.

13. The catheter in accordance with claim 12, wherein the distal end of the shaft (12) has a durometer that is less than that of the proximal end of the shaft.

## Patentansprüche

1. Intravaskulärer Katheter mit:
einem länglichen Schaft (12), der ein proximales Ende, ein distales Ende und eine distale Spitze mit einer formbaren Länge hat, wobei der längliche Schaft aufweist:
eine Innenlage (24);
eine zweite Schicht (26), die über der Innenlage (24) angeordnet ist, **dadurch gekennzeichnet, daß** die zweite Schicht (26) ein erstes Segment und ein zweites Segment aufweist, wobei sich das erste Segment zu einem distalen Abschluß (28) erstreckt und sich das zweite Segment vom distalen Abschluß (28) zu einem röntgendichten Markerband (40) erstreckt, das proximal zum distalen Ende des Schafts angeordnet ist, wobei der distale Abschluß (28) vom distalen Ende des Schafts um einen Abstand zurückgesetzt ist, der gleich oder größer als die formbare Länge ist;
eine dritte Schicht (30), die über der zweiten Schicht angeordnet ist, wobei die dritte Schicht (30) einen Einzelwendelbereich nahe dem distalen Ende des Schafts und
einen Mehrfachwendelbereich nahe dem proximalen Ende des Schafts aufweist; und
eine vierte Schicht (44), die über der dritten Schicht (30) angeordnet ist, wobei die vierte Schicht (44) ein proximales Ende und ein distales Ende aufweist, wobei der Härtewert am proximalen Ende größer als der Härtewert am distalen Ende ist.

2. Katheter nach Anspruch 1, wobei der distale Abschluß (28) etwa 4 mm vom distalen Ende des Schafts liegt.

3. Katheter nach Anspruch 2, wobei die Form der distalen Spitze wärmefixiert sein kann.

4. Katheter nach Anspruch 3, wobei die Form der distalen Spitze durch Dampf wärmefixiert sein kann.

5. Katheter nach Anspruch 1, wobei die Innenlage (24) Polytetrafluorethylen aufweist.

6. Katheter nach Anspruch 5, wobei die zweite Schicht (26) Polyetherblockamid aufweist.

7. Katheter nach Anspruch 6, wobei die dritte Schicht (30) eine Wendel aufweist.

8. Katheter nach Anspruch 7, wobei die Wendel Edelstahl aufweist.

9. Katheter nach Anspruch 7, wobei die Wendel Nickellegierung aufweist.

10. Katheter nach Anspruch 7, wobei die Wendel ein Nichteisenmetall aufweist.

11. Katheter nach Anspruch 7, wobei die vierte Schicht (44) Polyetherblockamid aufweist.

12. Katheter nach Anspruch 11, wobei das distale Ende des Schafts (12) einen Außendurchmesser hat, der kleiner als der Außendurchmesser des proximalen Endes des Schafts ist.

13. Katheter nach Anspruch 12, wobei das distale Ende des Schafts (12) einen Härtewert hat, der kleiner als der des proximalen Endes des Schafts ist.

## Revendications

1. Cathéter intravasculaire, comprenant :
une tige allongée (12) présentant une extrémité proximale, une extrémité distale, et une pointe distale présentant une longueur adaptable, la tige allongée comprenant :
une gaine interne (24) ;
une deuxième couche (26) disposée sur la gaine interne (24), **caractérisée en ce que** la deuxième couche (26) comprend un premier segment et un second segment, le premier segment s'étendant jusqu'à une terminaison distale (28) et le second segment s'étendant de la terminaison distale (28) à une bande de marqueur radio-opaque (40) disposée de manière proximale par rapport à l'extrémité distale de la tige, la terminaison distale (28) étant écartée de l'extrémité distale de la tige d'une distance supérieure ou égale à la longueur adaptable;
une troisième couche (30) disposée sur la deuxième couche, la troisième couche (30) comprenant une région à simple spirale à proximité de l'extrémité distale de la tige et une région à spirales multiples à proximité de l'extrémité proximale de la tige ; et
une quatrième couche (44) disposée sur la troisième couche (30), la quatrième couche (44) comprenant une extrémité proximale et une extrémité distale, dont la dureté d'après duromètre à l'extrémité proximale est supérieure à la dureté d'après duromètre à l'extrémité distale.

2. Cathéter selon la revendication 1, dans lequel la terminaison distale (28) est à environ 4 millimètres de l'extrémité distale de la tige.

3. Cathéter selon la revendication 2, dans lequel la forme de la pointe distale peut être thermodurcie.

4. Cathéter selon la revendication 3, dans lequel la forme de la pointe distale peut être thermodurcie par la vapeur.

5. Cathéter selon la revendication 1, dans lequel la gaine interne (24) comprend du polytétrafluoroéthylène.

6. Cathéter selon la revendication 5, dans lequel la deuxième couche (26) comprend un polyéther bloc amide.

7. Cathéter selon la revendication 6, dans lequel la troisième couche (30) comprend une spirale.

8. Cathéter selon la revendication 7, dans lequel la spirale comprend de l'acier inoxydable.

9. Cathéter selon la revendication 7, dans lequel la spirale comprend un alliage de nickel.

10. Cathéter selon la revendication 7, dans lequel la spirale comprend un métal non ferreux.

11. Cathéter selon la revendication 7, dans lequel la quatrième couche (44) comprend un polyéther bloc amide.

12. Cathéter selon la revendication 11, dans lequel l'extrémité distale de la tige (12) présente un diamètre extérieur qui est inférieur au diamètre extérieur de l'extrémité proximale de la tige.

13. Cathéter selon la revendication 12, dans lequel l'extrémité distale de la tige (12) présente une dureté d'après duromètre qui est inférieure à celle de l'extrémité proximale de la tige.
